(19) Europäisches Patentamt
European Patent Office
Office européen des brevets

(11) **EP 1 828 097 B1**

(12) **EUROPÄISCHE PATENTSCHRIFT**

(45) Veröffentlichungstag und Bekanntmachung des Hinweises auf die Patenterteilung:
**07.05.2008 Patentblatt 2008/19**

(21) Anmeldenummer: **05819382.2**

(22) Anmeldetag: **14.12.2005**

(51) Int Cl.:
**C07C 209/72** (2006.01)

(86) Internationale Anmeldenummer:
**PCT/EP2005/013403**

(87) Internationale Veröffentlichungsnummer:
**WO 2006/066762 (29.06.2006 Gazette 2006/26)**

(54) **VERFAHREN ZUR HERSTELLUNG VON 1,2-DIAMINO-3-METHYLCYCLOHEXAN UND/ODER 1,2-DIAMINO-4-METHYLCYCLOHEXAN**

METHOD FOR PRODUCING 1,2-DIAMINO-3-METHYLCYCLOHEXANE AND/OR 1,2-DIAMINO-4-METHYLCYCLOHEXANE

PROCEDE DE PRODUCTION DE 1,2-DIAMINO-3-METHYLCYCLOHEXANE ET / OU DE 1,2-DIAMINO-4-METHYLCYCLOHEXANE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **17.12.2004 DE 102004061608**

(43) Veröffentlichungstag der Anmeldung:
**05.09.2007 Patentblatt 2007/36**

(73) Patentinhaber: **BASF SE**
**67056 Ludwigshafen (DE)**

(72) Erfinder:
• **OSSWALD, Friederike**
**68163 Mannheim (DE)**
• **BRAUCH, Karl Heinz**
**68623 Lampertheim (DE)**
• **BÖTTCHER, Arnd**
**25050 Kuantan (MY)**
• **HENKELMANN, Jochem**
**68165 Mannheim (DE)**
• **VAN LAAR, Frederik**
**67117 Limburgerhof (DE)**
• **GERLACH, Till**
**67071 Ludwigshafen (DE)**

(56) Entgegenhaltungen:
**EP-A- 0 335 272          EP-A- 1 329 446**
**US-A- 3 636 108          US-A- 6 043 395**

EP 1 828 097 B1

**Beschreibung**

Die vorliegende Erfindung betrifft ein Verfahren zur Herstellung von 1,2-Diamino-3-methylcyclohexan und/oder 1,2-Diamino-4-methylcyclohexan durch Umsetzung von 2,3- und/oder 3,4-Diaminotoluol mit Wasserstoff bei erhöhtem Druck und erhöhter Temperatur in Gegenwart eines Rhodium-haltigen Heterogenkatalysators.

1,2-Diamino-methylcyclohexane, d.h. 1,2-Diamino-3-methylcyclohexan und 1,2-Diamino-4-methylcyclohexan, sind wichtige Ausgangsstoffe, insbesondere zur Herstellung von Härtern (Vernetzungsmitteln) für Epoxyharze. (Literatur: z.B. US-A-4,321,354 und J.W. Muskopf et al. "Epoxy Resins" in Ullmann's Encyclopedia of Industrial Chemistry, 6th Edition, Vol. 12, Seiten 285-303).

In der Patentliteratur wird die Kernhydrierung von aromatischen Diaminen ausführlich am Beispiel der 4,4'-Methylendianilin (MDA) zu 4,4'-Methylendicyclohexylamin (PACM) beschrieben. Dabei werden Toluoldiamine und deren Hydrierung allgemein oft mit erwähnt.

Beispielhaft seien diesbezüglich die folgenden Schutzrechte genannt:

US-A-3,450,759 , CA-B-892 636 (beide Mobay Chem. Comp.), US-A-3,636,108 (Du-Pont), DE-A-2 132 547 (BASF AG), JP-A-5 921 6852 (Nippon Kayaku), US-A-4,960,941, US-A-5,026,914 (beide Air Products), US-A-5,214,212 (Olin Corp.), US-A-5,981,801 (Korea Institute of Technology), US-A-5,973,207 (Air Products), US-A-6,075,167 (Olin Corp.), US-A-1,229,021 (Air Products).

Die Hydrierung der ortho-substituierten Toluoldiamine (2,3- und 3,4-Diaminotoluol) wird dabei nicht explizit beschrieben.

Im Patent US-A-3,450,759 wird gelehrt, dass eine Hydrierung der meta-Toluoldiamine (2,4/2,6-Toluoldiamine) erst nach vorheriger Abtrennung der ortho-Toluoldiamine möglich wird, da diese als Katalysatorgift wirken. Es wird davon ausgegangen, dass diese Verbindungen mit ihren benachbarten Aminofunktionen Chelat-Komplexe mit dem Aktivmetall bilden und dadurch die Oberfläche des Katalysators irreversibel belegen.

Im Patent US-A-5,973,207 wurde von Air Products ein verbesserter Hydrierprozess der meta-Toluoldiamine ohne vorherige Abtrennung der kleinen Mengen an ortho-Toluoldiaminen beschrieben.

Eine erstmals explizite Beschreibung der Hydrierung der vicinalen Toluoldiamine wurde von Air Products 2002 im Patent US-A-6,429,338 (Äquivalent: EP-A-1 329 446) veröffentlicht. Demnach wird die Hydrierung der Diamine durch Verknüpfung dreier wesentlicher Merkmale erreicht: Verwendung

    a) eines Rh-Katalysators,
    b) eines $C_{4-12}$-Dialkylethers als Lösungsmittel und
    c) einer semi-batch-Fahrweise, in der das Toluoldiamin während der Hydrierung langsam in den Autoklaven zugefahren wird.

Nachteilig kann hier das Zufahren der zu hydrierenden Substanz, d.h. diese semi-batch-Fahrweise sein. Damit kann ein gegenüber einer batch-Fahrweise erhöhter apparatetechnischer und/oder mess- und regelungstechnischer Aufwand verbunden sein, wie z.B. eine beheizte Vorlage und/oder eine beheizte Zudosierungsleitung für das Toluoldiamin.

Der vorliegenden Erfindung lag die Aufgabe zugrunde, ein verbessertes wirtschaftliches Verfahren zur Herstellung von 1,2-Diamino-methylcyclohexanen aufzufinden. Die Diamine 1,2-Diamino-3-methylcyclohexan und/oder 1,2-Diamino-4-methylcyclohexan sollten dabei in hoher Ausbeute, insbesondere Raum-Zeit-Ausbeute, Selektivität, Reinheit und/oder Farbqualität anfallen.

[Raum-Zeit-Ausbeuten werden angegeben in ‚Produktmenge / (Katalysatorvolumen ● Zeit)' (kg/($I_{Kat.}$ ● h)) und/oder ‚Produktmenge / (Reaktorvolumen ● Zeit)' (kg/($I_{Reaktor}$ ● h)].

Demgemäß wurde ein Verfahren zur Herstellung von 1,2-Diamino-3-methylcyclohexan und/oder 1,2-Diamino-4-methylcyclohexan durch Umsetzung von 2,3- und/oder 3,4-Diaminotoluol mit Wasserstoff bei erhöhtem Druck und erhöhter Temperatur in Gegenwart eines Rhodium-haltigen Heterogenkatalysators gefunden, welches dadurch gekennzeichnet ist, dass man eine Mischung enthaltend 2,3- und/oder 3,4-Diaminotoluol, einen Dialkylether und/oder alicyclischen Ether als Lösungsmittel und Ammoniak in einem Autoklav in Gegenwart des Katalysators vorlegt und anschließend unter Zufuhr von Wasserstoff hydriert.

Das erfindungsgemäße Verfahren stellt bevorzugt eine batch-Fahrweise (diskontinuierliche Fahrweise) und keine semi-batch-Fahrweise (semikontinuierliche Fahrweise) dar. D.h. das zu hydrierende 2,3- und/oder 3,4-Diaminotoluol wird komplett im Autoklav vorgelegt und es wird dem Autoklav bevorzugt kein 2,3- und/oder 3,4-Diaminotoluol während der Hydrierung zugeführt.

Erfindungsgemäß wurde die vorteilhafte Verfahrensweise in Gegenwart von Ammoniak erkannt. Durch die Verwendung von $NH_3$ als Additiv, ist es möglich, die Hydrierung vorteilhaft diskontinuierlich (batch-Fahrweise) durchzuführen. Der Katalysator bleibt auch bei höheren Toluoldiamin-Konzentrationen im Reaktionsmedium stabil und aktiv.

Das erfindungsgemäße Verfahren lässt sich wie folgt ausführen:

Der Einsatzstoff 2,3- und/oder 3,4-Diaminotoluol wird bevorzugt in einer Reinheit von $\geq$ 90 Gew.-%, insbesondere $\geq$ 98 Gew.-%, z.B. 98,2 bis 99,9 Gew.-%, eingesetzt. Solche Reinheiten können z.B. durch Destillation oder Rektifikation von kommerziell erhältlicher Ware erzielt werden.

Als Lösungsmittel wird bevorzugt ein $C_{4-12}$-Dialkylether und/oder $C_{3-12}$-alicyclischer Ether, insbesondere ein $C_{4-6}$-Dialkylether und/oder $C_{4-6}$-alicyclischer Ether, eingesetzt. Beispiele hierfür sind Methyl-tert.-butylether (MTBE), Diethylether (DEE), Di-n-propylether, Di-n-butylether, 1,2-Dimethoxyethan, 1,2-Diethoxyethan, Tetrahydrofuran (THF), 2-Methyl-THF, Tetrahydropyran, 1,3-Dioxepan, 1,4-Dioxan, 1,3-Dioxan und 1,3-Dioxolan. Besonders bevorzugt ist THF.

Bevorzugt wird eine 5 bis 50 Gew.-%ige, besonders 10 bis 40 Gew.-%ige, insbesondere 20 bis 25 Gew.-%ige, Lösung des Diaminotoluols oder der beiden Diaminotoluole (in Summe) im Lösungsmittel eingesetzt.

Als Lösungsmittel kann auch ein Gemisch von zwei oder mehr der genannten Dialkylether und/oder alicyclischen Ether eingesetzt werden.

Optional können im erfindungsgemäßen Hydrierverfahren weitere inerte Lösungsmittel, wie aliphatische oder cycloaliphatische Lösungsmittel, zugegen sein. Beispiele hierfür sind $C_{5-8}$-Kohlenwasserstoffe wie n-Pentan, n-Hexan und Cyclohexan.

Bevorzugt wird zusammen mit dem/den Diaminotoluol/en Lithiumhydroxid (LiOH), insbesondere 0,1 bis 5 Mol-% LiOH, ganz besonders 0,5 bis 1,5 Mol-% LiOH, besonders bevorzugt 0,8 bis 1,2 Mol-%, z.B. 1 Mol-%, LiOH, jeweils bezogen auf das Diaminotoluol oder die beiden Diaminotoluole (in Summe), eingesetzt.

In einer bevorzugten Ausführungsform wird die entsprechende Menge an LiOH als wässrige Lösung, z.B. als 5 bis 20 Gew.-%ige wässrige Lösung, eingesetzt.

Die Hydrierung wird in Gegenwart von Ammoniak durchgeführt. Bevorzugt setzt man 5 bis 500 Mol-%, insbesondere 250 bis 350 Mol-%, ganz besonders 280 bis 320 Mol-%, z.B. 300 Mol-%, Ammoniak, jeweils bezogen auf das Diaminotoluol oder die beiden Diaminotoluol (in Summe), ein.

Die Hydrierung erfolgt insbesondere bei Temperaturen im Bereich von 130 bis 220 °C, ganz besonders 140 bis 195 °C.

Der bei der Hydrierung angewandte Absolutdruck liegt insbesondere im Bereich von 100 bis 300 bar, ganz besonders 200 bis 280 bar.

Als Rhodium-haltige Heterogenkatalysatoren können marktübliche Rh-Katalysatoren eingesetzt werden. Der Katalysator kann auch nach dem Fachmann bekannten Verfahren hergestellt werden.

Beispiel für einen geeigneten kommerziell erhältlichen Katalysator ist:

5 % $Ru/Al_2O_3$ (Aldrich Produktnummern: 21,285-7; 37,971-9).

Bevorzugte Katalysatoren enthalten im Bereich von 1 bis 25 Gew.-%, insbesondere im Bereich von 2 bis 5 Gew.-%, Rhodium, bezogen auf das Trägermaterial.

Der Katalysator kann zusätzlich weitere Metalle, wie Ru, Pd, Ni, Fe, Ir und/oder Co, enthalten, z.B. in Mengen (in Summe) im Bereich von 0,01 bis 5 Gew.-%, insbesondere im Bereich von 0,1 bis 2,5 Gew.-%, bezogen auf das Trägermaterial.

Bevorzugte Katalysatoren enthalten als Trägermaterial kappa-, delta- und/oder gamma-Aluminiumoxid.

In einer besonderen Ausführungsform besteht das Trägermaterial aus kappa-, delta- und/oder gamma-Aluminiumoxid.

Besonders bevorzugtes Trägermaterial ist gamma-Aluminiumoxid. Dieses ist z.B. von BASF AG erhältlich (D10-10).

Ein ganz besonders bevorzugter Katalysator besteht aus gamma-Aluminiumoxid als Trägermaterial und Rhodium.

In einer besonderen Ausführungsform wird der eingesetzte Rhodium-haltige Katalysator zuvor mit Lithiumhydroxid behandelt. Diese Behandlung ist besonders dann vorteilhaft, wenn die Hydrierung in Abwesenheit von LiOH im vorgelegten Reaktionsgemisch durchgeführt wird.

Diese Behandlung des Katalysators mit LiOH kann nach dem Fachmann bekannten Verfahren erfolgen, z.B. durch Sättigen des Katalysators mit LiOH, z.B. 0,01 bis 5,0 Gew.-% LiOH (bez. auf das Trägermaterial), in Gegenwart eines geeigneten Lösungsmittels, z.B. Wasser. (EP-A1-913 388, US 6,429,338, US 3,636,108).

Als Reaktoren für das erfindungsgemäße Verfahren können zum Beispiel übliche Hochdruckautoklaven eingesetzt werden.

Nach der Durchführung des erfindungsgemäßen Verfahrens kann die Isolierung des Produkts bzw. der Produkte z.B. durch Destillation erfolgen.

In einer Ausführungsform kann das anfallende Verfahrensprodukt vorteilhaft ohne weitere Aufarbeitung als Vernetzungsmittel oder Komponente in einem Vernetzungsmittel für Epoxidharze eingesetzt werden.

Die mit dem erfindungsgemäßen Verfahren erzielbaren Umsätze an Diaminotoluol liegen im Bereich von $\geq 95$ %, insbesondere $\geq 99$ %, z.B. $\geq 96$ bis 99,9 % oder 99,5 bis 100 %, bei Selektivitäten (für die Bildung von 1,2-Diamino-3-methylcyclohexan und/oder 1,2-Diamino-4-methylcyclohexan) im Bereich von $\geq 80$ %, insbesondere $\geq 85$ %, z.B. 86 bis 99,5 % oder 90 bis 99 %.

Beispiele

Herstellung eines Rh/gamma-Al$_2$O$_3$-Katalysators (Katalysator A)

Es wurde eine Sprühtränklösung durch Lösen von Rhodiumtrichlorid (Degussa; Charge Nr. 26661, 37,9 % Rh enthaltend) in Wasser hergestellt, bis ein Volumen erhalten wurde, welches 30 % der Wasseraufnahmekapazität betrug. Diese Lösung wurde auf gamma-Al$_2$O$_3$ von BASF AG (D10-10; Wasseraufnahmekapazität: 5,8 ml Wasser pro 10 g D10-10) als Träger gesprüht und anschließend 12 h bei 120 °C unter Bewegung getrocknet.

Der getrocknete Katalysatorvorläufer wurde bei 300 °C für 4 h in einem WasserstoffStrom reduziert. Nach Abkühlen auf Raumtemperatur wurde der reduzierte Katalysator passiviert (Luft in N$_2$). Dabei wurde darauf geachtet, dass die Temperatur unter 40 °C blieb. Der so hergestellte Katalysator enthielt 3 Gew.-% Rh.

Hydrierung

a) Allgemeine Versuchsdurchführung

Der Einsatzstoff vicinales Toluoldiamin wurde von BASF Schwarzheide GmbH bezogen und war in einer Destillation über Kopf von dem schwerer siedenden Isomerengemisch 2,4/2,6-Toluoldiamin abgetrennt worden. Durch Luftkontakt während des Abfüllvorgangs entstand eine dunkelbraune Farbe des Feststoffs. Die Reinheit betrug 99,3 % (GC-Flächenprozent).

Die abgewogene Menge des vicinalen Toluoldiamins wurde in THF gelöst und mit einer bestimmten Menge an Katalysator (in Pulverform) zusammen in einen 0,3- (bzw. 1,2-) Liter-Autoklaven gegeben. Der Autoklav wurde verschlossen und es erfolgte eine Druckprüfung bis 250 bar mit Stickstoff. Nach Entspannen des Autoklaven wurde ein Druck von 50 bar Wasserstoff eingestellt und auf Reaktionstemperatur aufgeheizt. Nach Erreichen der Temperatur wurde mit Wasserstoff auf 250 bar aufgepresst. Die Hydrierzeit begann. Während der Reaktion abfallender Druck wurde durch Nachpressen von Wasserstoff ausgeglichen. Nach Beendigung der Hydrierzeit wurde der Autoklav entspannt und unter Stickstoff die Reaktionsmischung ausgetragen. Eine Probe wurde entnommen, der Katalysator mittels Filtration abgetrennt und das Filtrat über Gaschromatographie untersucht. Entstandene Nebenprodukte sind bezeichnet als

a) Monomer = Methylcyclohexylamin, entstanden durch Ammoniak-Abspaltung des Produkts und

b) Dimer = N,N-Bis(aminomethylcyclohexyl)amin und/oder N,N-Bis(aminomethylphenyl)amin, entstanden durch Kondensation zweier Produkt- bzw. E-duktmoleküle.

b) Zugabe von verschiedenen Zusätzen

Versuch A: keine Zusätze
Versuch B: Zugabe von Lithiumhydroxid
Versuch C: Zugabe von Ammoniak
Versuch D: Zugabe von Lithiumhydroxid und Ammoniak

150 ml einer 20 Gew.-%igen Lösung des vicinalen Toluoldiamins in THF, 1,5 g Katalysator A (3 Gew.-% Rh/Al$_2$O$_3$) und 0,6 g 10 Gew.-%ige wässrige Lithiumhydroxid-Lösung (Versuche B und D) bzw. 20 ml Ammoniak (Versuch C und D) wurden in einem 0,3-Liter-Autoklaven vorgelegt. Bei 180 °C und 250 bar wurde für 5 (Versuche B und D) bzw. für 4 Stunden (Versuch A) bzw. für 11 Stunden (Versuch C) hydriert.

Ergebnisse:

| Versuch | Edukt [%] | Produkt [%] | Diamin [%] | Monoamin [%] |
|---------|-----------|-------------|------------|--------------|
| A | 1 | 54 | 38 | 7 |
| B | < 0,1 | 81 | 11 | 8 |
| C | 5 | 65 | 19 | 11 |
| D | 0,2 | 89 | 4 | 7 |

Produkt = 3-Methylcyclohexan-1,2-diamin bzw. 4-Methylcyclohexan-1,2-diamin
Diamin = N,N-Bis(aminomethylcyclohexyl)amin und/oder N,N-Bis(aminomethylphenyl)-amin,
Monoamin = Methylcyclohexylamin

Die Hydrierung verlief ohne Zusatz von Additiven unselektiv, da in größerem Maße Kondensationsprodukte entstanden. In Anwesenheit von Ammoniak war der Reaktionsverlauf langsamer und selektiver. Lithiumhydroxid steigerte die Aktivität sowie die Selektivität des Katalysators. Die besten Ergebnisse bezüglich einer vollständigen und selektiven Reaktion wurden durch die Kombination der Zusätze Ammoniak und Lithiumhydroxid erzielt.

**Patentansprüche**

1. Verfahren zur Herstellung von 1,2-Diamino-3-methylcyclohexan und/oder 1,2-Diamino-4-methylcyclohexan durch Umsetzung von 2,3- und/oder 3,4-Diaminotoluol mit Wasserstoff bei erhöhtem Druck und erhöhter Temperatur in Gegenwart eines Rhodium-haltigen Heterogenkatalysators, **dadurch gekennzeichnet, dass** man eine Mischung enthaltend 2,3- und/oder 3,4-Diaminotoluol, einen Dialkylether und/oder alicyclischen Ether als Lösungsmittel und Ammoniak in einem Autoklav in Gegenwart des Katalysators vorlegt und anschließend unter Zufuhr von Wasserstoff hydriert.

2. Verfahren nach Anspruch 1, **dadurch gekennzeichnet, dass** die vorgelegte Mischung zusätzlich Lithiumhydroxid enthält.

3. Verfahren nach dem vorhergehenden Anspruch, **dadurch gekennzeichnet, dass** man das Lithiumhydroxid als wässrige Lösung einsetzt.

4. Verfahren nach einem der vorhergehenden Ansprüche, **dadurch gekennzeichnet, dass** man einen $C_{4-12}$-Dialkylether und/oder $C_{3-12}$-alicyclischen Ether als Lösungsmittel einsetzt.

5. Verfahren nach einem der Ansprüche 1 bis 3, **dadurch gekennzeichnet, dass** man Tetrahydrofuran (THF) als Lösungsmittel einsetzt.

6. Verfahren nach einem der vorhergehenden Ansprüche, **dadurch gekennzeichnet, dass** die Hydrierung bei einem Absolutdruck im Bereich von 100 bis 300 bar durchgeführt wird.

7. Verfahren nach einem der vorhergehenden Ansprüche, **dadurch gekennzeichnet, dass** die Hydrierung bei einer Temperatur im Bereich von 130 bis 220 °C durchgeführt wird.

8. Verfahren nach einem der vorhergehenden Ansprüche, **dadurch gekennzeichnet, dass** man eine 5 bis 50 Gew.-%ige Lösung des Diaminotoluols oder der beiden Diaminotoluole im Lösungsmittel einsetzt.

9. Verfahren nach einem der vorhergehenden Ansprüche, **dadurch gekennzeichnet, dass** man 5 bis 500 Mol-% Ammoniak bezogen auf das oder die beiden Diaminotoluol/e einsetzt.

10. Verfahren nach einem der Ansprüche 2 bis 9, **dadurch gekennzeichnet, dass** man 0,1 bis 5 Mol-% Lithiumhydroxid bezogen auf das oder die beiden Diaminotoluol/e einsetzt.

11. Verfahren nach einem der vorhergehenden Ansprüche, **dadurch gekennzeichnet, dass** der Heterogenkatalysator 1 bis 25 Gew.-% Rhodium bezogen auf das Trägermaterial enthält.

12. Verfahren nach einem der vorhergehenden Ansprüche, **dadurch gekennzeichnet, dass** der Heterogenkatalysator als Trägermaterial kappa-, delta- und/oder gamma-Aluminiumoxid enthält.

13. Verfahren nach einem der vorhergehenden Ansprüche, **dadurch gekennzeichnet, dass** der Heterogenkatalysator aus gamma-Aluminiumoxid als Trägermaterial und Rhodium besteht.

14. Verfahren nach einem der vorhergehenden Ansprüche, **dadurch gekennzeichnet, dass** der eingesetzte Heterogenkatalysator zuvor mit Lithiumhydroxid behandelt wurde.

15. Verfahren nach einem der vorhergehenden Ansprüche, **dadurch gekennzeichnet, dass** man 2,3- und/oder 3,4-Diaminotoluol in einer Reinheit von $\geq$ 90 Gew.-% einsetzt.

16. Verfahren nach einem der vorhergehenden Ansprüche, **dadurch gekennzeichnet, dass** eine batch-Fahrweise und keine semi-batch-Fahrweise angewendet wird.

**Claims**

1. A process for preparing 1,2-diamino-3-methylcyclohexane and/or 1,2-diamino-4-methylcyclohexane by reacting 2,3- and/or 3,4-diaminotoluene with hydrogen at elevated pressure and elevated temperature in the presence of a heterogeneous rhodium catalyst, which comprises initially charging a mixture comprising 2,3- and/or 3,4-diaminotoluene, a dialkyl ether and/or alicyclic ether as a solvent and ammonia in an autoclave in the presence of the catalyst and subsequently hydrogenating while supplying hydrogen.

2. The process according to claim 1, wherein the initially charged mixture additionally comprises lithium hydroxide.

3. The process according to the preceding claim, wherein the lithium hydroxide is used in the form of an aqueous solution.

4. The process according to the preceding claim, wherein a $C_{4-12}$-dialkyl ether and/or $C_{3-12}$-alicyclic ether is used as the solvent.

5. The process according to any of claims 1 to 3, wherein tetrahydrofuran (THF) is used as the solvent.

6. The process according to any of the preceding claims, wherein the hydrogenation is carried out at an absolute pressure in the range from 100 to 300 bar.

7. The process according to any of the preceding claims, wherein the hydrogenation is carried out at a temperature in the range from 130 to 220°C.

8. The process according to any of the preceding claims, wherein a from 5 to 50% by weight solution of the diaminotoluene or of the two diaminotoluenes in the solvent is used.

9. The process according to any of the preceding claims, wherein from 5 to 500 mol% of ammonia based on the diaminotoluene or the two diaminotoluenes is used.

10. The process according to any of claims 2 to 9, wherein from 0.1 to 5 mol% of lithium hydroxide based on the diaminotoluene or the two diaminotoluenes is used.

11. The process according to any of the preceding claims, wherein the heterogeneous catalyst comprises from 1 to 25% by weight of rhodium based on the support material.

12. The process according to any of the preceding claims, wherein the heterogeneous catalyst comprises kappa-, delta- and/or gamma-alumina as the support material.

13. The process according to any of the preceding claims, wherein the heterogeneous catalyst consists of gamma-alumina as the support material and rhodium.

14. The process according to any of the preceding claims, wherein the heterogeneous catalyst used has been treated beforehand with lithium hydroxide.

15. The process according to any of the preceding claims, wherein 2,3- and/or 3,4-diaminotoluene is used in a purity of ≥ 90% by weight.

16. The process according to any of the preceding claims, wherein a batchwise method and not a semibatchwise method is employed.

**Revendications**

1. Procédé de fabrication de 1,2-diamino-3-méthylcyclohexane et/ou de 1,2-diamino-4-méthylcyclohexane par réaction de 2,3- et/ou 3,4-diaminotoluène avec de l'hydrogène à pression supérieure et à température augmentée en présence d'un catalyseur hétérogène contenant du rhodium, **caractérisé en ce qu'**on met d'abord dans un autoclave en présence du catalyseur un mélange contenant du 2,3- et/ou 3,4-diaminotoluène, un dialkyléther et/ou un éther alicyclique comme solvant et de l'ammoniac, puis on hydrogène sous apport d'hydrogène.

2. Procédé selon la revendication 1, **caractérisé en ce que** le mélange préalable contient en plus de l'hydroxyde de lithium.

3. Procédé selon la revendication précédente, **caractérisé en ce qu'**on emploie l'hydroxyde de lithium sous forme de solution aqueuse.

4. Procédé selon l'une des revendications précédentes, **caractérisé en ce qu'**on utilise un dialkyléther en $C_4$-$C_{12}$ et/ou un éther alicyclique en $C_3$-$C_{12}$ comme solvant.

5. Procédé selon l'une des revendications 1 à 3, **caractérisé en ce qu'**on utilise du tétrahydrofurane (THF) comme solvant.

6. Procédé selon l'une des revendications précédentes, **caractérisé en ce que** l'hydrogénation se fait à pression absolue dans l'intervalle compris entre 100 et 300 bars.

7. Procédé selon l'une des revendications précédentes, **caractérisé en ce que** l'hydrogénation se fait à une température comprise dans l'intervalle entre 130 et 220 °C.

8. Procédé selon l'une des revendications précédentes, **caractérisé en ce qu'**on utilise une solution à 5 à 50 % en poids du diamine toluène ou des deux diamine toluènes en solvant.

9. Procédé selon l'une des revendications précédentes, **caractérisé en ce qu'**on utilise 5 à 500 % en mole d'ammoniac sur base du ou des deux diamine toluènes.

10. Procédé selon l'une des revendications 2 à 9, **caractérisé en ce qu'**on utilise 0,1 à 5 % en mole d'hydroxyde de lithium, sur base du ou des deux diamine toluènes.

11. Procédé selon l'une des revendications précédentes, **caractérisé en ce que** le catalyseur hétérogène contient 1 à 25 % en poids de rhodium, sur base de la matière de support.

12. Procédé selon l'une des revendications précédentes, **caractérisé en ce que** le catalyseur hétérogène contient comme matière de support de l'oxyde de kappa-, delta- et/ou gamma-aluminium.

13. Procédé selon l'une des revendications précédentes, **caractérisé en ce que** le catalyseur hétérogène est constitué d'oxyde de gamma-aluminium comme matière de support et de rhodium.

14. Procédé selon l'une des revendications précédentes, **caractérisé en ce que** le catalyseur hétérogène utilisé a été préalablement traité avec de l'hydroxyde de lithium.

15. Procédé selon l'une des revendications précédentes, **caractérisé en ce qu'**on utilise du 2,3- et/ou du 3,4-diami-

notoluène dans une pureté $\geq$ 90 % en poids.

16. Procédé selon l'une des revendications précédentes, **caractérisé en ce qu'**on applique un mode de marche par lots et pas de mode de marche par demi-lot.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4321354 A
- US 3450759 A
- CA 892636 B
- US 3636108 A
- DE 2132547 A
- JP 59216852 A
- US 4960941 A
- US 5026914 A
- US 5214212 A
- US 5981801 A
- US 5973207 A
- US 6075167 A
- US 1229021 A
- US 6429338 A
- EP 1329446 A
- EP 913388 A1
- US 6429338 B

**In der Beschreibung aufgeführte Nicht-Patentliteratur**

- Epoxy Resins. **J.W. MUSKOPF et al.** Ullmann's Encyclopedia of Industrial Chemistry. vol. 12, 285-303